# EUROPEAN PATENT APPLICATION

(11) **EP 3 067 420 A1**
(43) Date of publication of application: **14.09.2016**
(21) Application number: 16154588.4
(22) Date of filing: 10.11.2011
(51) Int. Cl.: C12N 15/113, A61P 31/06

(54) **ANTISENSE ANTIBACTERIAL COMPOUNDS AND METHODS**

(30) Priority: 12.11.2010 US 412971 P; 07.12.2010 US 420636 P
(62) Divisional of application: 11784908.3
(71) Applicant: Sarepta Therapeutics, Inc., Bothell, Washington 98021 (US)
(72) Inventor: IVERSEN, Patrick, L., Corvallis, OR 97330 (US)
(74) Representative: Schiweck, Weinzierl & Koch

(57) **Abstract**

Antibacterial antisense compounds and method of using the same for treating *a Mycobacterium tuberculosis* infection in a mammalian host are disclosed. The compounds include an antisense oligonucleotide conjugated to a carrier peptide that significantly enhances the antibacterial activity of the oligonucleotide. The antisense oligonucleotides contain 10-20 nucleotide bases and have a targeting nucleic acid sequence complementary to a target sequence containing or within 20 bases, in a downstream direction, of the translational start codon of a bacterial mRNA that encodes a bacterial protein essential for bacterial replication, where the compound binds to a target mRNA with a Tₘ of between 45° to 60°C. The carrier peptide is an arginine-rich peptide containing between 6 and 14 amino acids. Antisense compounds that target host factor genes that facilitate *Mycobacterium tuberculosis* infection are also provided, as are methods of using these compounds to treat *Mycobacterium tuberculosis* infections, alone or in combination with other therapies.

SEQUENCE LISTING

<110>  Sarepta Therapeutics, Inc.

<120>  ANTISENSE ANTIBACTERIAL COMPOUNDS AND METHODS

<130>  SIP14770PCTEPD1

<140>  Divisional application based on EP11784908.3
<141>  2011-11-10

<150>  US 61/420,636
<151>  2010-12-07

<150>  US 61/412,971
<151>  2010-11-12

<160>  42

<170>  PatentIn version 3.5

<210>  1
<211>  11
<212>  DNA
<213>  Artificial

<220>
<223>  Antisense targeting sequence leuD1

<400>  1
gtgaaaggct t                                                            11


<210>  2
<211>  11
<212>  DNA
<213>  Artificial

<220>
<223>  Antisense targeting sequence leuD2

<400>  2
cttccatgct g                                                            11


<210>  3
<211>  11
<212>  DNA
<213>  Artificial

<220>
<223>  Antisense targeting sequence leuD3

<400>  3
ctgatcccct a                                                            11


<210>  4
<211>  11
<212>  DNA
<213>  Artificial

<220>

<223>  Antisense targeting sequence mgtC1

<400>  4
gtcagcgttt g                                                            11


<210>  5
<211>  11
<212>  DNA
<213>  Artificial

<220>
<223>  Antisense targeting sequence mgtC2

<400>  5
tttgcatgac g                                                            11


<210>  6
<211>  11
<212>  DNA
<213>  Artificial

<220>
<223>  Antisense targeting sequence mtgC3

<400>  6
acgccgagtc c                                                            11


<210>  7
<211>  11
<212>  DNA
<213>  Artificial

<220>
<223>  Antisense targeting sequence pirG

<400>  7
cggttcggca c                                                            11


<210>  8
<211>  11
<212>  DNA
<213>  Artificial

<220>
<223>  Antisense targeting sequence Rv0194-1

<400>  8
gcaattcgtg c                                                            11


<210>  9
<211>  11
<212>  DNA
<213>  Artificial

<220>
<223>  Antisense targeting sequence Rv0194-2

<400>  9

tgcgcattcg c                                                            11

<210>  10
<211>  11
<212>  DNA
<213>  Artificial

<220>
<223>  Antisense targeting sequence Rv0194-3

<400>  10
cgccatatcc t                                                            11

<210>  11
<211>  11
<212>  DNA
<213>  Artificial

<220>
<223>  Antisense targeting sequence pcaA1

<400>  11
gagctgcacg g                                                            11

<210>  12
<211>  11
<212>  DNA
<213>  Artificial

<220>
<223>  Antisense targeting sequence pcaA2

<400>  12
acggacatac c                                                            11

<210>  13
<211>  11
<212>  DNA
<213>  Artificial

<220>
<223>  Antisense targeting sequence pcaA3

<400>  13
ccgattgagc c                                                            11

<210>  14
<211>  11
<212>  DNA
<213>  Artificial

<220>
<223>  Antisense targeting sequence cma1-1

<400>  14
cagctcgtcg g                                                            11

<210>  15
<211>  11
<212>  DNA
<213>  Artificial

<220>
<223>  Antisense targeting sequence cma1-2

<400>  15
cgggcattgg g                                                            11


<210>  16
<211>  11
<212>  DNA
<213>  Artificial

<220>
<223>  Antisense targeting sequence cma1-3

<400>  16
gggatagcgt a                                                            11


<210>  17
<211>  21
<212>  DNA
<213>  Artificial

<220>
<223>  Host Gene Targeting Sequence IL-10E4SA

<400>  17
ggagaaatcg atgctgaaga a                                                 21


<210>  18
<211>  20
<212>  DNA
<213>  Artificial

<220>
<223>  Host Gene Targeting Sequence SOCS1

<400>  18
ctggttgcgt gctaccatcc                                                   20


<210>  19
<211>  21
<212>  DNA
<213>  Artificial

<220>
<223>  Host Gene Targeting Sequence SOCS3


<220>
<221>  misc_feature
<222>  (17)..(17)
<223>  n = Inosine

<220>

<221>  misc_feature
<222>  (19)..(19)
<223>  n = Inosine

<400>  19
gccgctaccg catcccngng a                                                21


<210>  20
<211>  21
<212>  DNA
<213>  Artificial

<220>
<223>  Host Gene Targeting Sequence STAT3

<400>  20
tggcttctca agatacctgc t                                                21


<210>  21
<211>  20
<212>  DNA
<213>  Artificial

<220>
<223>  Host Gene Targeting Sequence IL-27-AUG

<400>  21
gtcacctggc ccatgtcagg                                                  20


<210>  22
<211>  20
<212>  DNA
<213>  Artificial

<220>
<223>  Host Gene Targeting Sequence IL-27E3SA

<400>  22
caatcgagat tcagcctgag                                                  20


<210>  23
<211>  20
<212>  DNA
<213>  Artificial

<220>
<223>  Host Gene Targeting Sequence TGF-beta


<220>
<221>  misc_feature
<222>  (13)..(15)
<223>  n = Inosine

<400>  23
gagggcggca tgnnngaggc                                                  20


<210>  24

<211>  11
<212>  PRT
<213>  Artificial

<220>
<223>  Peptide Sequence (RFF)3;CP0407


<220>
<221>  MOD_RES
<222>  (10)..(10)
<223>  Xaa = 6-aminohexanoic acid

<220>
<221>  MOD_RES
<222>  (11)..(11)
<223>  Xaa = beta-alanine

<400>  24

Arg Phe Phe Arg Phe Phe Arg Phe Phe Xaa Xaa
1               5                   10


<210>  25
<211>  12
<212>  PRT
<213>  Artificial

<220>
<223>  Peptide Sequence RTR


<220>
<221>  MOD_RES
<222>  (11)..(11)
<223>  Xaa = 6-aminohexanoic acid

<220>
<221>  MOD_RES
<222>  (12)..(12)
<223>  Xaa = beta-alanine

<400>  25

Arg Thr Arg Thr Arg Phe Leu Arg Arg Thr Xaa Xaa
1               5                   10


<210>  26
<211>  12
<212>  PRT
<213>  Artificial

<220>
<223>  Peptide Sequence RFFR


<220>
<221>  MOD_RES
<222>  (11)..(11)
<223>  Xaa = 6-aminohexanoic

<220>
<221>  MOD_RES
<222>  (12)..(12)
<223>  Xaa = beta-alanine

<400>  26

Arg Phe Phe Arg Phe Phe Arg Phe Phe Arg Xaa Xaa
1               5                   10


<210>  27
<211>  12
<212>  PRT
<213>  Artificial

<220>
<223>  Peptide Sequence KTR


<220>
<221>  MOD_RES
<222>  (11)..(11)
<223>  Xaa = 6-aminohexanoic acid

<220>
<221>  MOD_RES
<222>  (12)..(12)
<223>  Xaa = beta-alanine

<400>  27

Lys Thr Arg Thr Lys Phe Leu Lys Lys Thr Xaa Xaa
1               5                   10


<210>  28
<211>  11
<212>  PRT
<213>  Artificial

<220>
<223>  Peptide Sequence KFF


<220>
<221>  MOD_RES
<222>  (10)..(10)
<223>  Xaa = 6-aminohexanoic

<220>
<221>  MOD_RES
<222>  (11)..(11)
<223>  Xaa = beta-alanine

<400>  28

Lys Phe Phe Lys Phe Phe Lys Phe Phe Xaa Xaa
1               5                   10


<210>  29
<211>  12

<212>  PRT
<213>  Artificial

<220>
<223>  Peptide Sequence KFFK


<220>
<221>  MOD_RES
<222>  (11)..(11)
<223>  Xaa = 6-aminohexanoic acid

<220>
<221>  MOD_RES
<222>  (12)..(12)
<223>  Xaa = beta-alanine

<400>  29

Lys Phe Phe Lys Phe Phe Lys Phe Phe Lys Xaa Xaa
1               5                   10


<210>  30
<211>  8
<212>  PRT
<213>  Artificial

<220>
<223>  Peptide Sequence(RFF)2


<220>
<221>  MOD_RES
<222>  (7)..(7)
<223>  Xaa = 6-aminohexanoic acid

<220>
<221>  MOD_RES
<222>  (8)..(8)
<223>  Xaa = beta-alanine

<400>  30

Arg Phe Phe Arg Phe Phe Xaa Xaa
1               5


<210>  31
<211>  9
<212>  PRT
<213>  Artificial

<220>
<223>  Peptide Sequence (RFF)2R


<220>
<221>  MOD_RES
<222>  (8)..(8)
<223>  Xaa = 6-aminohexanoic acid

<220>

<221>  MOD_RES
<222>  (9)..(9)
<223>  Xaa = beta-alanine

<400>  31

Arg Phe Phe Arg Phe Phe Arg Xaa Xaa
1               5


<210>  32
<211>  10
<212>  PRT
<213>  Artificial

<220>
<223>  Peptide Sequence RAhx


<220>
<221>  MOD_RES
<222>  (2)..(3)
<223>  Xaa = 6-aminohexanoic acid

<220>
<221>  MOD_RES
<222>  (5)..(6)
<223>  Xaa = 6-aminohexanoic acid

<220>
<221>  MOD_RES
<222>  (8)..(9)
<223>  Xaa = 6-aminohexanoic acid

<220>
<221>  MOD_RES
<222>  (10)..(10)
<223>  Xaa = beta-alanine

<400>  32

Arg Asn Asn Arg Xaa Xaa Arg Xaa Xaa Xaa
1               5                   10


<210>  33
<211>  14
<212>  PRT
<213>  Artificial

<220>
<223>  Peptide Sequence (RAhxR)4;P007


<220>
<221>  MOD_RES
<222>  (2)..(2)
<223>  Xaa = 6-aminohexanoic acid

<220>
<221>  MOD_RES
<222>  (5)..(5)
<223>  Xaa = 6-aminohexanoic acid

<220>
<221>  MOD_RES
<222>  (8)..(8)
<223>  Xaa = 6-aminohexanoic acid

<220>
<221>  MOD_RES
<222>  (11)..(11)
<223>  Xaa = 6-aminohexanoic acid

<220>
<221>  MOD_RES
<222>  (13)..(13)
<223>  Xaa = 6-aminohexanoic acid

<220>
<221>  MOD_RES
<222>  (14)..(14)
<223>  Xaa = beta-alanine

<400>  33

Arg Xaa Arg Arg Xaa Arg Arg Xaa Arg Arg Xaa Arg Xaa Xaa
1               5                   10


<210>  34
<211>  11
<212>  DNA
<213>  Artificial

<220>
<223>  PPMO Sequence P007leuD1

<400>  34
gtgaaaggct t                                                            11


<210>  35
<211>  11
<212>  DNA
<213>  Artificial

<220>
<223>  Antisense targeting sequence Rv0477c-1

<400>  35
agggctttca t                                                            11


<210>  36
<211>  11
<212>  DNA
<213>  Artificial

<220>
<223>  Antisense targeting sequence Rv0477c-2

<400>  36
caccagggct t                                                            11

<210>  37
<211>  11
<212>  DNA
<213>  Artificial

<220>
<223>  Antisense targeting sequence Rv2958c-1

<400>  37
attcccgcag c                                                            11


<210>  38
<211>  11
<212>  DNA
<213>  Artificial

<220>
<223>  Antisense targeting sequence Rv2958c-2

<400>  38
agcacatcga a                                                            11


<210>  39
<211>  12
<212>  DNA
<213>  Artificial

<220>
<223>  Antisense targeting sequence Rv2958c-3

<400>  39
gaaaagtgcg ct                                                           12


<210>  40
<211>  11
<212>  DNA
<213>  Artificial

<220>
<223>  Antisense targeting sequence Rv2957-1

<400>  40
tttcgtctgc a                                                            11


<210>  41
<211>  11
<212>  DNA
<213>  Artificial

<220>
<223>  Antisense targeting sequence Rv2957-2

<400>  41
gtctgcacca t                                                            11


<210>  42
<211>  14
<212>  PRT

<213>  Artificial

<220>
<223>  PPMO Sequence P007leuD1



<220>
<221>  MOD_RES
<222>  (1)..(1)
<223>  Xaa = beta-alanine

<220>
<221>  MOD_RES
<222>  (2)..(2)
<223>  Xaa = 6-aminohexanoic acid

<220>
<221>  MOD_RES
<222>  (4)..(4)
<223>  Xaa = 6-aminohexanoic acid

<220>
<221>  MOD_RES
<222>  (7)..(7)
<223>  Xaa = 6-aminohexanoic acid

<220>
<221>  MOD_RES
<222>  (10)..(10)
<223>  Xaa = 6-aminohexanoic acid

<220>
<221>  MOD_RES
<222>  (13)..(13)
<223>  Xaa = 6-aminohexanoic acid

<400>  42

Xaa Xaa Arg Xaa Arg Arg Xaa Arg Arg Xaa Arg Arg Xaa Arg
1               5                   10

## Description

### CROSS-REFERENCE(S) TO RELATED APPLICATION(S)

This Application claims the benefit under 35 U.S.C. § 119(e) of U.S. Provisional Application No. 61/412,971, filed November 12, 2010 and U.S. Provisional Application No. 61/420,636, filed December 7, 2010, where these Provisional Applications are incorporated herein by reference in their entireties.

### SEQUENCE LISTING

The Sequence Listing associated with this application is provided in text format in lieu of a paper copy, and is hereby incorporated by reference into the specification. The name of the text file containing the Sequence Listing is 120178_490PC_SEQUENCE_LISTING.txt, created November 10, 2011, and being about 11 KB. The text file is being submitted electronically via EFS-Web.

### FIELD OF THE INVENTION

The present invention relates to oligonucleotide analog compounds that are antisense to *Mycobacterium tuberculosis* genes and to selected mammalian host genes, and methods of using such compounds to inhibit bacterial growth, *e.g*., for treating an infected mammalian subject.

### BACKGROUND OF THE INVENTION

Currently, there are several types of antibiotic compounds in use against bacterial pathogens and these compounds act through a variety of anti-bacterial mechanisms. For example, beta-lactam antibiotics, such as penicillin and cephalosporin, act to inhibit the final step in peptidoglycan synthesis. Glycopeptide antibiotics, including vancomycin and teichoplanin, inhibit both transglycosylation and transpeptidation of muramyl-pentapeptide, again interfering with peptidoglycan synthesis. Other well-known antibiotics include the quinolones, which inhibit bacterial DNA replication, inhibitors of bacterial RNA polymerase, such as rifampin, and inhibitors of enzymes in the pathway for production of tetrahydrofolate, including the sulfonamides.

Some classes of antibiotics act at the level of protein synthesis. Notable among these are the aminoglycosides, such as kanamycin and gentamycin. This class of compounds targets the bacterial 30S ribosome subunit, preventing the association with the 50S subunit to form functional ribosomes. Tetracyclines, another important class of antibiotics, also target the 30S ribosome subunit, acting by preventing alignment of aminoacylated tRNA's with the corresponding mRNA codon. Macrolides and lincosamides, another class of antibiotics, inhibit bacterial synthesis by binding to the 50S ribosome subunit, and inhibiting peptide elongation or preventing ribosome translocation.

Despite impressive successes in controlling or eliminating bacterial infections by antibiotics, the widespread use of antibiotics both in human medicine and as a feed supplement in poultry and livestock production has led to drug resistance in many pathogenic bacteria. Antibiotic resistance mechanisms can take a variety of forms. One of the major mechanisms of resistance to beta lactams, particularly in Gram-negative bacteria, is the enzyme beta-lactamase, which renders the antibiotic inactive. Likewise, resistance to aminoglycosides often involves an enzyme capable of inactivating the antibiotic, in this case by adding a phosphoryl, adenyl, or acetyl group. Active efflux of antibiotics is another way that many bacteria develop resistance. Genes encoding efflux proteins, such as the tetA, tetG, tetL, and tetK genes for tetracycline efflux, have been identified. A bacterial target may develop resistance by altering the target of the drug. For example, the so-called penicillin binding proteins (PBPs) in many beta-lactam resistant bacteria are altered to inhibit the critical antibiotic binding to the target protein. Resistance to tetracycline may involve, in addition to enhanced efflux, the appearance of cytoplasmic proteins capable of competing with ribosomes for binding to the antibiotic. For those antibiotics that act by inhibiting a bacterial enzyme, such as for sulfonamides, point mutations in the target enzyme may confer resistance.

The appearance of antibiotic resistance in many pathogenic bacteria, including *Mycobacterium tuberculosis*, in many cases involving multi-drug resistance (MDR), has raised the specter of a pre-antibiotic era in which many bacterial pathogens are simply untreatable by medical intervention. There are two main factors that could contribute to this scenario. The first is the rapid spread of resistance and multi-drug resistance genes across bacterial strains, species, and genera by conjugative elements, the most important of which are self-transmissible plasmids. The second factor is a lack of current research efforts to find new types of antibiotics, due in part to the perceived investment in time and money needed to find new antibiotic agents and bring them through clinical trials, a process that may require a 20-year research effort in some cases.

In addressing the second of these factors, some drug-discovery approaches that may accelerate the search for new antibiotics have been proposed. For example, efforts to screen for and identify new antibiotic compounds by high-throughput screening have been reported, but to date no important lead compounds have been discovered by this route. Another approach is to target host genes that facilitate bacterial escape from the immune system including genes involved in the IL-10 signaling pathway (see e.g., PCT Application No. US08/088339).

According to World Health Organization ("WHO") estimates, nearly two billion people (one-third of the world's population) are thought to be infected with *M*. *tuberculosis* (MTb), while three to five million are permanently disabled due to *M*. *leprae* infection. While the majority of those infected with MTb do not show clinical signs of disease, each year about nine million people develop active TB, and nearly two million die from it [Dye et al., 1999]. In the US the estimated prevalence of TB infection was 4.2% in 1999-2000, representing approximately 9.5 million TB infected persons among the civilian population [Khan et al 2008]. TB is the leading cause of death from a single bacterial infection, and the leading cause of mortality in persons infected with HIV.

Despite aggressive treatment protocols in use for more than a century, MTb infection remains one of the most serious threats to world health, particularly in developing and third-world nations. A key reason that the pathogen remains a significant health risk for the human population relates to its remarkable ability to persist for long periods of time in the host body, even in the face of immunity, chemotherapy, and/or antimicrobial therapy. The emergence of MDR strains of the bacterium has not only exacerbated successful treatment, but also increased the arsenal of potential bioterrorism agents.

Further, despite significant advances in antimicrobial therapies over the past four decades, contemporary treatment regimens for the eradication of TB and other pathogenic Mycobacteria are largely ineffective. The WHO's current treatment guidelines for TB-infected individuals require a minimum six- to nine-month multidrug treatment regimen that includes a combination of at least four of the so-called "first-line" antibiotics: isoniazid ("INH"), rifampin ("RFP"), rifabutin ("RFB"), rifapentine ("RPT"), pyrazinamide ("PZA") and ethambutol ("EMB"). Unfortunately, these lengthy treatments are both expensive and suffer from poor patient compliance, a problem exacerbated by the toxic side effects that often occur during prolonged treatment regimens.

There is thus a growing need for new antibiotics that (i) are not subject to the principal types of antibiotic resistance currently hampering antibiotic treatment of MTb infection, (ii) can be developed rapidly and with some reasonable degree of predictability as to target-bacteria specificity, (iii) can also be designed for broad-spectrum activity, (iv) are effective at low doses and (v) show few side effects.

Several approaches that involve antisense agents designed to block the expression of bacterial resistance genes or to target bacterial RNA targets, such as the rRNA in the 30S ribosomal subunit, have been proposed (Rahman, Summerton et al. 1991; Good and Nielsen 1998). In general, these approaches have been successful only in a limited number of cases, or have required high antisense concentrations (*e.g*., (Summerton, Stein et al. 1997), or the requirement that the treated cells show high permeability for antibiotics (Good and Nielsen 1998; Geller, Deere et al. 2003). Recent improvements include the use of antisense oligomers targeted to essential genes and conjugated to cell penetrating peptides as described in PCT publication Nos. WO2008/008113 and WO2007/009094. Greenburg et al (Greenberg, Marshall-Batty et al. 2010) have shown the utility of the latter approach in targeting multi-drug resistant members of the *Burkholderia cepacia* complex. Despite these promising results, there remains a need to identify improved antisense targets for a variety of pathogenic bacteria including extensively multi-drug resistant *Mycobacterium tuberculosis.*

In addition, novel approaches are needed such as those that incorporate targeting host genes to overcome MTb escape from the host immune response. These strategies can provide many advantages including broad spectrum activity and control of multi-drug resistant MTb.

### SUMMARY OF THE INVENTION

The invention includes, in one aspect, an antibacterial antisense compound for use in treating a *Mycobacterium tuberculosis* (MTb) infection in a mammalian host. The compound includes a substantially uncharged antisense oligonucleotide having between 10-20 bases and a targeting sequence of at least 10 contiguous bases complementary to a target region of the infecting bacteria's mRNA for leuD, mgtC, pirG, Rv0194, Rv0477c, Rv2958c, and Rv2957, pcaA, acpP, gyrA and cma genes, where the target region contains the translational start codon of the bacterial mRNA, or a sequence that is within 20 bases of the translational start codon, and where oligonucleotide binds to the mRNA to form a heteroduplex having a Tₘ of at least 45°C, thereby to inhibit replication of the bacteria. Exemplary oligonucleotides that target the above disclosed genes are listed below as SEQ ID NOs: 1 -16 and 35-41.

Another aspect of the invention is to target host genes involved in MTb escape from either the innate or adaptive host immune response. Antisense compounds targeted to genes that encode the IL-10 signaling pathway, the STAT3 gene, IL-27 and/or TGFbeta are provided and listed below as SEQ ID NOs: 16 - 23.

Optionally included in the compound, and conjugated to the oligonucleotide at either the 5' or 3' terminus, is an arginine-rich carrier protein coupled to the oligonucleotide at the peptide's C terminus, and preferably represented by the peptide sequence (RXX)ₙ- or (RXR)ₙ, where X is an uncharged amino acid selected from the group consisting of alanine, valine, leucine, isoleucine, serine, glycine threonine, phenyalanine and tryptophan, and n= 2 to 4. X can also be a non-natural amino acid consisting of, for example, β-alanine, 6-aminohexanoic acid or with the general structure of -CO-(CH₂)ₙ-CHR-NH-, where n is 2 to 7 and R is H. For example, when n is 5 and R is H, X is a 6-aminohexanoic acid subunit, abbreviated herein as Ahx.

In exemplary embodiments, the carrier peptide has the sequence (RFF)ₙ or (RFF)ₙR, where n = 2 or 3. The carrier peptide may be linked at its C-terminus to one end of the oligonucleotide, e.g., the 5'-end, through a one- or two-amino acid linker, such as the linker is AhxβAla, where Ahx is 6-aminohexanoic acid and βAla is β-alanine. In a preferred embodiment, the carrier peptide has the sequence (RAhxR)ₙ-, where n = 4. The carrier peptide may be linked at its C-terminus to one end of the oligonucleotide, e.g., the 5'-or 3'-end, through a one- or two-amino acid linker, such as the linker AhxβAla, where Ahx is 6-aminohexanoic acid and βAla is β-alanine. The carrier peptide has the ability, when conjugated to the 3'-end of the oligonucleotide, to enhance the anti-bacterial activity of the oligonucleotide, as measured by inhibition in bacterial growth *in vivo* over a two-day period, by a factor of at least 10². Exemplary peptides of the invention are listed below as SEQ IS NOs: 24-33.

The oligonucleotide of the compound may be composed of morpholino subunits and phosphorus-containing intersubunit linkages joining a morpholino nitrogen of one subunit to a 5' exocyclic carbon of an adjacent subunit. The morpholino subunits in the oligonucleotide may be joined by phosphorodiamidate linkages, in accordance with the structure: 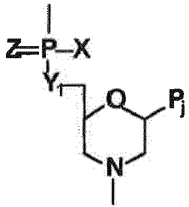 where Y₁=O, Z=O, Pj is a purine or pyrimidine base-pairing moiety effective to bind, by base-specific hydrogen bonding, to a base in a polynucleotide, and X is alkyl, alkoxy, thioalkoxy, amino or alkyl amino, including dialkylamino (designated as PMO or PPMO for an unconjugated or carrier peptide conjugated morpholino oligomer containing phosphorodiamidate linkages, respectively).

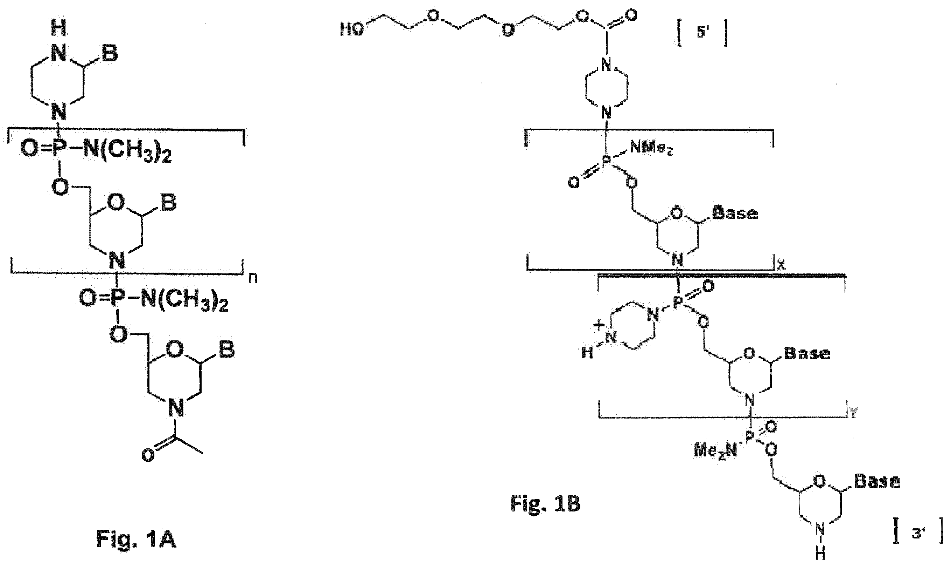

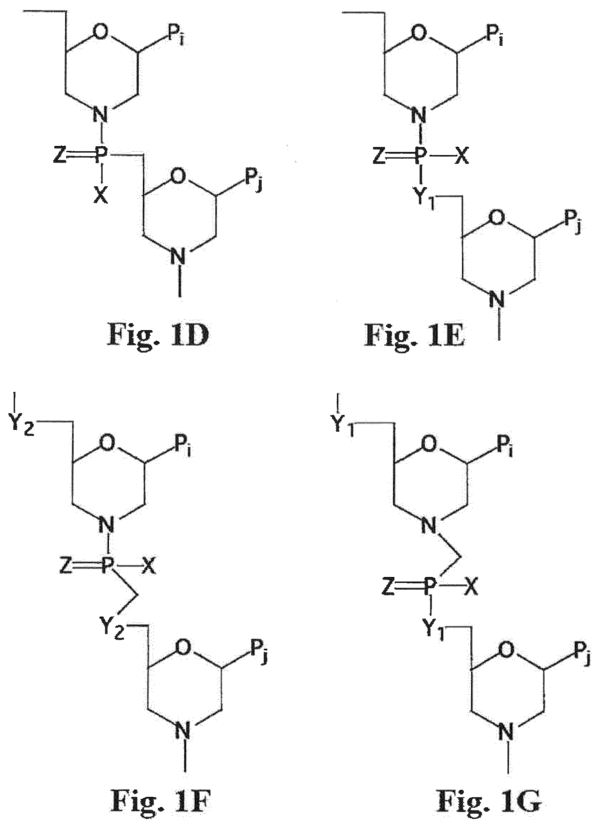

The compound may also be composed of morpholino subunits linked with the uncharged linkages described above and interspersed with linkages that are positively charged at physiological pH (designated as PMO+ or P-PMO+). The total number of positively charged linkages is at least 1, preferably at least 2, 3, 4, 5, or 6, and no more than half of the total number of linkages, typically between 2 and 5 positively charged linkages. The positively charged linkages may have the structure above, where X is 1-piperazine. The structure shown in Figure 1B is an exemplary positively charged linkage type. The targeting sequence of the oligonucleotide may be complementary to a target sequence containing or within 20 bases, in a downstream direction, of the translational start codon of a MTb mRNA that encodes one of the following genes: ribosomal protein S10 (rpsJ); leuD; mgtC; pirG; pcaA; cma1; Rv0194; Rv0477c; Rv2958c; and Rv2957. In various embodiments the targeting sequence may be complementary to at least ten contiguous bases that incorporate the AUG start codon of an mRNA sequence encoding the above listed genes.

In accordance with another aspect of the invention, the compound is used in treating a bacterial infection, specifically a multiple drug resistant *Mycobacterium tuberculosis* infection, by administering a therapeutically effective amount of the compound to a mammalian host.

Accordingly, certain embodiments include a substantially uncharged antisense morpholino oligonucleotide, composed of morpholino subunits and phosphorus-containing intersubunit linkages joining a morpholino nitrogen of one subunit to a 5'-exocyclic carbon of an adjacent subunit, and having a) 10-20 nucleotide bases; b) a targeting sequence of at least 10 bases in length directed against a bacterial mRNA that encodes an essential protein from *Mycobacterium tuberculosis* (MTb), wherein said targeting sequence is complementary to a target sequence containing the translational start codon of said bacterial mRNA or to a target sequence containing 20 bases downstream of the translational start codon of said bacterial mRNA; and c) a Tm, when hybridized to the target sequence, of between about 45°C to 65°C.

In some embodiments, the morpholino subunits are joined by phosphorodiamidate linkages in accordance with the structure: 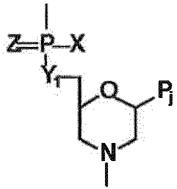 where Y₁=O, Z=O, Pj is a purine or pyrimidine base-pairing moiety effective to bind, by base-specific hydrogen bonding, to a base in a polynucleotide, and X is alkyl, alkoxy, thioalkoxy, or alkyl amino. In certain embodiments, at least 2 and no more than half of the total number of intersubunit linkages are positively charged. In particular embodiments, X=NR₂, and each R is independently hydrogen or methyl, and for the positively charged linkages, X is 1-piperazine.

In certain embodiments, the essential MTb protein is selected from *rpsJ*, *leuD*, *mgtC*, *pirG*, *pcaA*, *cma1*, *Rv0194, Rv0477c*, *Rv2958c, Rv2957, gyrA*, and *acpP*. In specific embodiments, the targeting sequence comprises the nucleotide sequence set forth in at least one of SEQ ID NOS:1-16 or 35-41. In some embodiments, the oligonucleotide is conjugated to an arginine-rich polypeptide that enhances the uptake of the compound into host cells. In particular embodiments, the arginine-rich polypeptide is selected from the group consisting of SEQ ID NOS:24-34.

Also included are methods of reducing replication of *Mycobacterium tuberculosis* (MTb), comprising contacting an MTb bacteria or an MTb-infected host cell with an anti-MTb antisense morpholino oligonucleotide described herein. In certain embodiments, the bacteria or host cell is in a subject, and the method comprises administering the antisense oligonucleotide to the subject. In some embodiments, the MTb is a multidrug-resistant MTb (MDR-MTb) or an extensively-drug resistant MTb (XDR-MTb).

Also included are methods of reducing replication of *Mycobacterium tuberculosis* (MTb), comprising contacting an MTb-infected host cell with an antisense morpholino oligonucleotide, composed of morpholino subunits and phosphorus-containing intersubunit linkages joining a morpholino nitrogen of one subunit to a 5'-exocyclic carbon of an adjacent subunit, and having a) 10-20 nucleotide bases, b) a targeting sequence of at least 10 bases in length directed against a mammalian host mRNA that encodes IL-10, SOCS1, SOCS3, STAT3, IL-27, orTGF-beta, wherein said targeting sequence is complementary to (i) a target sequence containing the translational start codon of said mRNA, (ii) a target sequence containing 20 bases downstream of the translational start codon of said mRNA, or (iii) a target sequence containing the 50 bases surrounding a splice donor or a splice acceptor site of said mRNA; and c) a Tm, when hybridized to the target sequence, of between about 45°C to 65°C. In specific embodiments, the targeting sequence comprises the nucleotide sequence set forth in at least one of SEQ ID NOS:17-23. In particular embodiments, the oligonucleotide is conjugated to an arginine-rich polypeptide that enhances the uptake of the compound into host cells. In certain embodiments, the arginine-rich polypeptide is selected from the group consisting of SEQ ID NOS:24-34.

In certain embodiments, the host cell is in a subject, and the method comprises administering the antisense oligonucleotide to the subject. Also included are methods which further comprise administering separately or concurrently an anti-MTb antibiotic, an anti-MTb antisense oligonucleotide described herein, or both. In some embodiments,the anti-MTb antibiotic is selected from at least one of ethambutol, isoniazid, pyrazinamide, rifampicin, streptomycin, amikacin, kanamycin, capreomycin, viomycin, enviomycin, ciprofloxacin, levofloxacin, moxifloxacin, ethioniamid, prothionamide, cycloserine, p-aminosalicylic acid, rifabutin, clarithromycin, linezolid, thioacetamide, thioacetazone, and thioridazine.

Also included are pharmaceutical composition comprising an anti-MTb antisense oligonucleotide described herein, and a pharmaceutically acceptable carrier. In certain embodiments, the pharmaceutical composition further comprises an anti-host gene antisense oligonucleotide described herein.

These and other objects and features of the claimed subject matter will become more fully apparent when the following detailed description is read in conjunction with the accompanying drawings.

### BRIEF DESCRIPTION OF THE FIGURES

Figure 1A shows an exemplary morpholino oligomer structure with a phosphorodiamidate linkage;
Figure 1B shows a morpholino oligomer as in Figure 1A, but where the backbone linkages contain one positively charged group in the form of a (piperazino) phosphorodiamidate linkage;

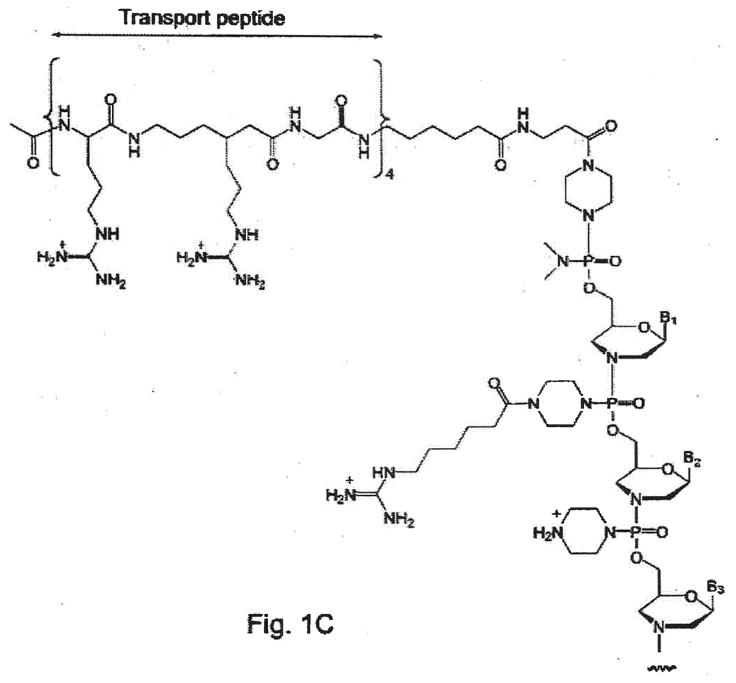

Figure 1C shows a conjugate of an arginine-rich peptide and an antisense oligomer, in accordance with one embodiment of the invention;
Figures 1D-G show the repeating subunit segment of exemplary morpholino oligonucleotides, designated D through G.

### DETAILED DESCRIPTION

### I. Definitions

The terms below, as used herein, have the following meanings, unless indicated otherwise:
As used herein, the terms "compound", "agent", "oligomer" and "oligonucleotide" may be used interchangeably with respect to the antisense oligonucleotides of the claimed subject matter. As used herein, the terms "antisense oligonucleotide" and "oligonucleotide" or "antisense compound" or "oligonucleotide compound " are used interchangeably and refer to a sequence of subunits, each having a base carried on a backbone subunit composed of ribose or other pentose sugar or morpholino group, and where the backbone groups are linked by intersubunit linkages (the majority of which are uncharged) that allow the bases in the compound to hybridize to a target sequence in an RNA by Watson-Crick base pairing, to form an RNA:oligonucleotide heteroduplex within the target sequence. The oligonucleotide may have exact sequence complementarity to the target sequence or near complementarity. Such antisense oligonucleotides are designed to block or inhibit translation of the mRNA containing the target sequence, and may be said to be "directed to" a sequence with which it hybridizes. Exemplary structures for antisense oligonucleotides for use in the claimed subject matter include the β-morpholino subunit types shown in Figs. 1A-C. It will be appreciated that a polymer may contain more than one linkage type.

The term "oligonucleotide" or "antisense oligonucleotide" also encompasses an oligonucleotide having one or more additional moieties conjugated to the oligonucleotide, e.g., at its 3'- or 5'- end, such as a polyethyleneglycol moiety or other hydrophilic polymer, e.g., one having 10-100 monomeric subunits, which may be useful in enhancing solubility, or a moiety such as a lipid or peptide moiety that is effective to enhance the uptake of the compound into target bacterial cells and/or enhance the activity of the compound within the cell, e.g., enhance its binding to a target polynucleotide.

The term "target sequence" refers to a portion of the target RNA against which the oligonucleotide or antisense agent is directed, that is, the sequence to which the oligonucleotide will hybridize by Watson-Crick base pairing of a complementary sequence. In certain embodiments, the target sequence may be a contiguous region of the translation initiation region of a bacterial gene of a host gene, or may be composed of the splice acceptor or splice donor sites of a host gene.

The term "targeting sequence" or "antisense targeting sequence" refers to the sequence in an oligonucleotide or other antisense agent that is complementary (meaning, in addition, substantially complementary) to the target sequence in the RNA genome. The entire sequence, or only a portion, of the antisense compound may be complementary to the target sequence. For example, in an oligonucleotide having 20-30 bases, about 6, 7, 8, 9, 10, 11, 12, 13, 14, 15, 16, 17, 18, 19, 20, 21, 22, 23, 24, 25, 26, 27, 28, or 29 may be targeting sequences that are complementary to the target region. Typically, the targeting sequence is formed of contiguous bases, but may alternatively be formed of non-contiguous sequences that when placed together, e.g., from opposite ends of the oligonucleotide, constitute sequence that spans the target sequence.

The target and targeting sequences may be selected such that binding of the antisense compound is to a region within; 45 bases surrounding the AUG start codons of the bacterial or host gene mRNA and/or the 50 bases surrounding the splice donor or acceptor sites of a mammalian host gene subject to alternative splicing. In certain embodiments, the target region may comprise both the AUG codon and the bases surrounding or contributing to the splice donor site of the host gene mRNA, such as a polypyrimidine tract or lariat-forming sequence. In certain embodiments, using a single antisense oligomer to target both the AUG start codon and the proximal splice donor sequences (*e.g*., polypyrimidine tract) of the host gene mRNA may provide synergistic effects with regard to reducing target protein expression, reducing bacterial replication, or both.

A carrier peptide conjugated to an antisense oligonucleotide, *e.g*., by covalent linkage between the peptide's C terminal end and the 5'- or 3'-end of the oligonucleotide, is separately named, *i.e*., not included within the term "oligonucleotide." The carrier peptide and covalently attached antisense oligonucleotide are also referred to herein as a conjugate or conjugate compound. More generally, a "peptide-conjugated morpholino antisense oligonucleotide" is a morpholino antisense oligonucleotide conjugated at either its 5' or 3' termini to an arginine-rich peptide carrier.

By "arginine-rich carrier peptide" is meant that the carrier peptide has at least 2, and preferably 2-4 (8-12) arginine residues, each preferably separated by one or more uncharged, hydrophobic residues, and preferably containing 6-14 amino acid residues. Exemplary arginine rich peptides are listed as SEQ ID NOS: 24 - 33. In exemplary embodiments, the carrier peptide has the sequence (RXX)ₙ, where X is an uncharged amino acid selected from the group consisting of alanine, valine, leucine, isoleucine, serine, threonine, phenyalanine, tyrosine, and tryptophan, and n= 2 or 3. In preferred embodiments, the carrier peptide has the form (RFF)ₙ or (RFF)ₙR, where n = 2 or 3. The carrier peptide may be linked at its C-terminus to one end of the oligonucleotide, e.g., the 5'-end, through a one- or two-amino acid linker, such as the linker is AhxβAla, where Ahx is 6-aminohexanoic acid and βAla is β-alanine, and where the linker forms part of the carrier peptide. Also in an exemplary embodiment, the carrier peptide has the sequence (RAhxR)ₙ, where n = 4. The carrier peptide may be linked at its C-terminus to one end of the oligonucleotide, *e.g*., the 5'- or 3'-end, through a one- or two-amino acid linker, such as the linker AhxβAla, where Ahx is 6-aminohexanoic acid and βAla is β-alanine.

Figure 1A shows an exemplary morpholino oligomer structure with a phosphorodiamidate linkage;

Figure 1B shows a morpholino oligomer as in Figure 1A, but where the backbone linkages contain one positively charged group in the form of a (piperazino) phosphorodiamidate linkage;

Figure 1C shows a conjugate of an arginine-rich peptide and an antisense morpholino oligomer including three different intersubunit linkages ;

Figures 1D-G show the repeating subunit segment of exemplary morpholino oligonucleotides, designated D through G.

As used herein, a "morpholino oligomer" or "morpholino oligonucleotide" refers to an antisense oligonucleotide having a backbone which supports bases capable of hydrogen bonding to natural polynucleotides, *e.g*., DNA or RNA, is composed of morpholino subunit structures of the form shown in Figures 1B and 1C, where (i) the structures are linked together by phosphorous-containing linkages, one to three atoms long, joining the morpholino nitrogen of one subunit to the 5' exocyclic carbon of an adjacent subunit, and (ii) Pᵢ and Pⱼ, Base or Bₙ are purine or pyrimidine base-pairing moieties effective to bind, by base-specific hydrogen bonding, to a base in a polynucleotide.

This preferred aspect of the claimed subject matter is illustrated in Figure 1A, which shows two such subunits joined by a phosphorodiamidate linkage. Morpholino oligonucleotides (including antisense oligonucleotides) are detailed, for example, in co-owned U.S. Pat. Nos. 5,698,685, 5,217,866, 5,142,047, 5,034,506, 5,166,315, 5,185,444, 5,521,063, and 5,506,337, and PCT Appn. Nos. PCT/US07/11435 and US2008/012804, all of which are incorporated herein by reference. Exemplary morpholino oligonucleotides with charged backbone linkages and/or modified terminal groups, including antisense oligonucleotides, are detailed in PCT application No. US2007/011435; co-pending U.S. Provisional Application No. 61/349,783; and co-pending U.S. Provisional Application No. 61/361,878, each of which is incorporated by reference in its entirety.

As used herein, a "nuclease-resistant" oligonucleotide molecule (oligonucleotide) is one whose backbone is not susceptible to nuclease cleavage of a phosphodiester bond. Exemplary nuclease resistant antisense oligonucleotides are oligonucleotide analogs, such as phosphorothioate and phosphate-amine DNA (pnDNA), both of which have a charged backbone, and methyl-phosphonate, and morpholino oligonucleotides, all of which may have uncharged backbones.

As used herein, an antisense oligonucleotide "specifically hybridizes" to a target polynucleotide if the oligonucleotide hybridizes to the target under physiological conditions, with a Tm greater than 37°C. As will be seen below, the antisense oligonucleotides of the claimed subject matter have a preferred Tm values with respect to their target mRNAs of at least 45° C, typically between 50°- 60°C or greater.

The "Tm" of an oligonucleotide compound, with respect to its target mRNA, is the temperature at which 50% of a target sequence hybridizes to a complementary polynucleotide. Tm is determined under standard conditions in physiological saline, as described, for example, in Miyada C.G. and Wallace R.B. 1987. Oligonucleotide hybridization techniques.Methods Enzymol. 154:94-107. Polynucleotides are described as "complementary" to one another when hybridization occurs in an antiparallel configuration between two single-stranded polynucleotides. A double-stranded polynucleotide can be "complementary" to another polynucleotide, if hybridization can occur between one of the strands of the first polynucleotide and the second. Complementarity (the degree that one polynucleotide is complementary with another) is quantifiable in terms of the proportion of bases in opposing strands that are expected to form hydrogen bonds with each other, according to generally accepted base-pairing rules.

As used herein, a first sequence or targeting sequence is an "antisense sequence" with respect to a second sequence or target sequence if a polynucleotide whose sequence is the first sequence specifically binds to, or specifically hybridizes with, the second polynucleotide sequence under physiological conditions.

As used herein, a "base-specific intracellular binding event involving a target RNA" refers to the sequence specific binding of an oligonucleotide to a target RNA sequence inside a cell. For example, a single-stranded polynucleotide can specifically bind to a single-stranded polynucleotide that is complementary in sequence.

As used herein, "nuclease-resistant heteroduplex" refers to a heteroduplex formed by the binding of an antisense oligonucleotide to its complementary target, which is resistant to *in vivo* degradation by ubiquitous intracellular and extracellular nucleases.

As used herein, "essential bacterial genes" are those genes whose products play an essential role in an organism's functional repertoire as determined using genetic footprinting or other comparable techniques to identify gene essentiality.

An agent is "actively taken up by bacterial cells" when the agent can enter the cell by a mechanism other than passive diffusion across the cell membrane. The agent may be transported, for example, by "active transport", referring to transport of agents across a mammalian cell membrane by *e.g.* an ATP-dependent transport mechanism, or by "facilitated transport", referring to transport of antisense agents across the cell membrane by a transport mechanism that requires binding of the agent to a transport protein, which then facilitates passage of the bound agent across the membrane. For both active and facilitated transport, the oligonucleotide compound preferably has a substantially uncharged backbone, as defined below.

As used herein, the terms "modulating expression" and "antisense activity" relative to an oligonucleotide refers to the ability of an antisense oligonucleotide to either enhance or reduce the expression of a given protein by interfering with the expression, or translation of RNA. In the case of reduced protein expression, the antisense oligonucleotide may directly block expression of a given gene, or contribute to the accelerated breakdown of the RNA transcribed from that gene.

As used herein, the term "inhibiting bacterial growth, refers to blocking or inhibiting replication and/or reducing the rate of replication of bacterial cells in a given environment, for example, in an infective mammalian host.

By "enhance" or "enhancing," or "increase" or "increasing," or "stimulate" or "stimulating," refers generally to the ability of one or antisense compounds or compositions to produce or cause a greater physiological response (*i.e.*, downstream effects) in a cell or a subject, as compared to the response caused by either no antisense compound or a control compound. An "increased" or "enhanced" amount is typically a "statistically significant" amount, and may include an increase that is 1.1, 1.2, 2, 3, 4, 5, 6, 7, 8, 9, 10, 15, 20, 30, 40, 50 or more times (*e.g*., 500, 1000 times) (including all integers and decimal points in between and above 1), *e.g.,* 1.5, 1.6, 1.7. 1.8, etc.) the amount produced by no antisense compound (the absence of an agent) or a control compound.

The term "reduce" or "inhibit" may relate generally to the ability of one or more antisense compounds of the invention to "decrease" a relevant physiological or cellular response, such as a symptom of a disease or condition described herein, as measured according to routine techniques in the diagnostic art. Relevant physiological or cellular responses (*in vivo* or *in vitro*) will be apparent to persons skilled in the art, and may include reductions in the symptoms or pathology of MTb infection, or reductions in bacterial replication or bacterial load. A "decrease" in a response may be "statistically significant" as compared to the response produced by no antisense compound or a control composition, and may include a 1%, 2%, 3%, 4%, 5%, 6%, 7%, 8%, 9%, 10%, 11%, 12%, 13%, 14%, 15%, 16%, 17%, 18% , 19%, 20%, 25%, 30%, 35%, 40%, 45%, 50%, 55%, 60%, 65%, 70%, 75%, 80%, 85%, 90%, 95%, or 100% decrease, including all integers in between.

As used herein, the term "pathogenic bacterium," or "pathogenic bacteria," or "pathogenic bacterial cells," refers to bacterial cells capable of infecting and causing disease in a mammalian host, as well as producing infection-related symptoms in the infected host, such as fever or other signs of inflammation, intestinal symptoms, respiratory symptoms, dehydration, and the like.

As used herein, the terms "Gram-negative pathogenic bacteria" or "Gram-negative bacteria" refer to the phylum of proteobacteria, which have an outer membrane composed largely of lipopolysaccharides. All proteobacteria are gram negative, and include, but are not limited to *Escherichia coli, Salmonella,* other *Enterobacteriaceae, Pseudomonas, Burkholderi, Moraxella, Helicobacter, Stenotrophomonas, Bdellovibrio,* acetic acid bacteria, and *Legionella.* Other notable groups of gram negative bacteria include *Haemophilus influenzae,* the cyanobacteria, spirochaetes, green sulfur and green non-sulfur bacteria. The pathogenic capability of gram negative bacteria is usually associated with components of the bacterial cell wall, in particular the lipopolysaccharide (also known as LPS or endotoxin) layer.

As used herein, the terms "Gram-positive pathogenic bacteria" or "Gram-positive bacteria" refer to those bacteria that are stained dark blue or violet by Gram staining, in contrast to Gram-negative bacteria, which cannot retain the stain, instead taking up the counterstain and appearing red or pink. The stain is caused by a high amount of peptidoglycan in the cell wall, which typically, but not always, lacks the secondary membrane and lipopolysaccharide layer found in Gram-negative bacteria. Gram-positive bacteria include many well-known genera such as *Bacillus, Listeria, Staphylococcus, Streptococcus, Enterococcus,* and *Clostridium.* It has also been expanded to include the *Mollicutes,* and bacteria such as *Mycoplasma,* which lack cell walls and so cannot be stained by Gram, but are derived from such forms. *Mycobacteria* (including *M*. *tuberculosis)* are considered acid-fast Gram-positive bacteria due to the lack of an outer cell membrane.

As used herein, "effective amount" or "therapeutically effective amount" or "growth-inhibiting amount" relative to an antisense oligonucleotide refers to the amount of antisense oligonucleotide administered to a mammalian subject, either as a single dose or as part of a series of doses and which is effective to inhibit bacterial replication in an infected host, by inhibiting translation of a selected bacterial target nucleic acid sequence. The ability to block or inhibit bacterial replication in an infected host may be evidenced by a reduction in infection-related symptoms.

As used herein "treatment" of an individual or a cell is any type of intervention used in an attempt to alter the natural course of the individual or cell. Treatment includes, but is not limited to, administration of e.g., a pharmaceutical composition, and may be performed either prophylactically, or subsequent to the initiation of a pathologic event or contact with an etiologic agent.

The terms "positively charged" and "cationic" refer to the predominant state of a chemical moiety at near-neutral pH, e.g. about 5 to 9. As described herein, a positively charged (cationic) phosphorodiamidate linkage comprises a 1-piperazine ring pendant to the phosphorus atom, as shown in Figures 1B and 1C.

A "substantially uncharged", phosphorus containing backbone in an oligonucleotide analog is one in which a majority of the subunit linkages, *e.g*., between 50-100%, are uncharged at physiological pH, and contain a single phosphorous atom, For example, the backbone may contain only uncharged linkages or 1 cationic linkage per every 3-10 linkages.

### II. Exemplary oligonucleotide backbones

Examples of nonionic linkages that may be used in oligonucleotide analogs are shown in Figures 1A and 1B. In these figures, B or Base represents a purine or pyrimidine base-pairing moiety effective to bind, by base-specific hydrogen bonding, to a base in a polynucleotide, preferably selected from adenine, cytosine, guanine, thymidine, uracil and inosine. Suitable backbone structures include carbonate (2A, R=O) and carbamate (2A, R=NH₂) linkages (Mertes and Coats 1969; Gait, Jones et al. 1974); alkyl phosphonate and phosphotriester linkages (2B, R=alkyl or -O-alkyl)(Lesnikowski, Jaworska et al. 1990); amide linkages (2C) (Blommers, Pieles et al. 1994); sulfone and sulfonamide linkages (2D, R1, R2 = CH₂); and a thioformacetyl linkage (2E) (Cross, Rice et al. 1997). The latter is reported to have enhanced duplex and triplex stability with respect to phosphorothioate antisense compounds (Cross, Rice et al. 1997).

A preferred oligonucleotide structure employs morpholino-based subunits bearing base-pairing moieties as illustrated in Figs. 1A-1D, joined by uncharged linkages, as described above. Especially preferred is a substantially uncharged phosphorodiamidate-linked morpholino oligonucleotide containing a cationic linkage every 3 to 10 linkages, such as illustrated in Figure 1B and 1C. Morpholino oligonucleotides, including antisense oligonucleotides, are detailed, for example, in (Summerton and Weller 1997) and in co-owned U.S. Patent Nos. 5,698,685, 5,217,866, 5,142,047, 5,034,506, 5,166,315, 5,185, 444, 5,521,063, 5,506,337 and PCT Appn. Nos. PCT/US07/11435 and US08/012804, all of which are incorporated herein by reference.

Important properties of the morpholino-based subunits include: 1) the ability to be linked in a oligomeric form by stable, uncharged or positively charged backbone linkages; 2) the ability to support a nucleotide base (*e.g.* adenine, cytosine, guanine, thymidine, uracil and inosine) such that the polymer formed can hybridize with a complementary-base target nucleic acid, including target RNA, Tm values above about 45°C in relatively short oligonucleotides (*e.g.,* 10-15 bases); 3) the ability of the morpholino oligonucleotide to be actively or passively transported into bacterial cells; and 4) the ability of the morpholino oligonucleotide:RNA heteroduplex to resist RNAse degradation.

### A. Exemplary antisense oligonucleotides

Exemplary backbone structures for antisense oligonucleotides of the claimed subject matter include the β-morpholino subunit types shown in Figs. 1A-1C, each linked by an uncharged or positively charged, phosphorus-containing subunit linkage..

The antisense compounds can be prepared by stepwise solid-phase synthesis, employing methods detailed in the references cited above. In some cases, it may be desirable to add additional chemical moieties to the antisense compound, *e.g.,* to enhance pharmacokinetics or to facilitate capture or detection of the compound. Such a moiety may be covalently attached, typically to a terminus of the oligomer, according to standard synthetic methods. For example, addition of a polyethyleneglycol moiety or other hydrophilic polymer, *e.g*., one having 10-100 monomeric subunits, may be useful in enhancing solubility. One or more charged groups, *e.g*., anionic charged groups such as an organic acid, may enhance cell uptake. A reporter moiety, such as fluorescein or a radiolabeled group, may be attached for purposes of detection. Alternatively, the reporter label attached to the oligomer may be a ligand, such as an antigen or biotin, capable of binding a labeled antibody or streptavidin. In selecting a moiety for attachment or modification of an antisense oligomer, it is generally of course desirable to select chemical compounds of groups that are biocompatible and likely to be tolerated by a subject without undesirable side effects.

### B. Antibacterial antisense oligonucleotides

In addition to the structural features described above, the antisense compound of the claimed subject matter contains no more than 20 nucleotide bases, and has a targeting nucleic acid sequence (the sequence which is complementary to the target sequence) of no fewer than 10 contiguous bases. For instance, certain antisense compounds consist of about 10-20 (*e.g*., 10, 11, 12, 13, 14, 15, 16, 17, 18, 19, 20) nucleotide bases, and have a targeting sequence of about 10, 11, 12, 13, 14, 15, 16, 17, 18, 19, or 20 contiguous bases that are complementary to the target sequence (*e.g*., the AUG codon, splice site). In certain embodiments, the targeting sequence is complementary to a target sequence containing or within 15 bases, in a downstream direction, of the translational start codon of a bacterial mRNA that encodes a bacterial protein essential for bacterial replication. The compound has a Tₘ, when hybridized with the target sequence, of at least about 45°C, typically between about 50° to 60°C, although the Tm may be higher, *e.g.,* 65°C. The selection of bacterial targets, and bacterial mRNA target sequences and complementary targeting sequences are considered in the two sections below.

The antisense compound is optionally covalently conjugated to an 8 to 12 residue arginine- rich peptide at either the 5'- or 3'-end of the antisense oligomer of the type described above.

### III. Bacterial targets,

This section considers a number of bacterial targets, including pathogenic bacteria, and specific bacterial protein targets against which the antisense compound can be directed.

### A. Bacterial Targets

*Mycobacterium tuberculosis* (MTb) is considered a Gram positive, acid fast bacterium which is the causative agent of tuberculosis, a sometimes crippling and deadly disease. Tuberculosis is on the rise globally and the leading cause of death from a single infectious disease with a current death rate of three million people per year. It can affect several organs of the human body, including the brain, the kidneys and the bones, however, tuberculosis most commonly affects the lungs.

In the United States, approximately ten million individuals are infected with *Mycobacterium tuberculosis*, as indicated by positive skin tests, with approximately 26,000 new cases of active disease each year. The increase in tuberculosis (TB) cases in the US has been associated with HIV/AIDS, homelessness, drug abuse and immigration of persons with active infections. Current treatment programs for drug-susceptible TB involve taking two or four drugs (*e.g*., isoniazid, rifampin, pyrazinamide, ethambutol or streptomycin), for a period of from six to nine months, because all of the MTb cannot be destroyed by a single drug. In addition, the observation of drug-resistant and multiple drug resistant strains of MTb is on the rise.

### B. Target proteins

The antisense oligomers of the claimed subject matter are designed to hybridize to a region of a bacterial mRNA that encodes an essential bacterial gene. Exemplary genes include, but are not limited to, those required for cell division, cell cycle proteins, lipid biosynthesis, nucleic acid replication and ion transport. Any essential bacterial gene can be targeted once a gene's sequence is determined. One approach to determining which genes in an organism are essential is to use genetic footprinting techniques as described (Gerdes, Scholle et al. 2003). In this report, 620 *E. coli* genes were identified as essential and 3,126 genes as dispensable for growth under culture conditions for robust aerobic growth. Evolutionary context analysis demonstrated that a significant number of essential *E. coli genes* are preserved throughout the bacterial kingdom, especially the subset of genes for key cellular processes such as DNA replication, cell division and protein synthesis.

In general, the target for modulation of gene expression using the antisense methods of the claimed subject matter comprises an mRNA expressed during active bacterial growth or replication, such as an mRNA sequence transcribed from one of the following genes including, but not limited to, ribosomal protein S10 (*rpsJ*), *leuD, mgtC, pirG*, *pcaA*, *cma1, Rv0194, Rv0477c, Rv2958c,* and *Rv2957* genes. Additional targets include genes involved in lipid biosynthesis (*e.g. acp*P) and replication (*e.g. gyr*A) as described previously (see e.g., PCT publication Nos. WO2008/008113 and WO2007/009094).

*LeuD* encodes the small subunit of isopropylmalate isomerase that catalyzes the isomerization between 2-isopropylmalate and 3-isopropylmalate in leucine biosynthesis.

*MgtC* encodes a protein that mediates magnesium transport (import) into the cytosol.

*PcaA* encodes a protein that is required for cording and mycolic acid cyclopropanation ring synthesis in the cell wall.

*Cma1* encodes the cyclopropane-fatty-acyl-phospholipid synthase 1 enzyme.

*Rv0194* encodes an ABC transporter of the ATP-binding protein class and plays a key role in antibiotic efflux in drug-resistant MTb.

*GyrA* refers to subunit A of the bacterial gyrase enzyme, and the gene therefore. Bacterial gyrase is one of the bacterial DNA topoisomerases that control the level of supercoiling of DNA in cells and is required for DNA replication.

*AcpP* encodes acyl carrier protein, an essential cofactor in lipid biosynthesis. The fatty acid biosynthetic pathway requires that the heat stable cofactor acyl carrier protein binds intermediates in the pathway. The antisense oligonucleotides of the present invention can be complementary to a target sequence containing or within 15 bases, in a downstream direction, of the translational start codon of a bacterial mRNA that encodes any of the above-mentioned bacterial genes.

### C. Selection of oligomer target sequences and lengths

As noted above, the claimed subject matter derives from the discovery that oligomeric antisense compounds having at least 10 and up to 20 bases complementary to a bacterial RNA target, e.g., at or just downstream (within 5, 10, 15, 20 bases) of the AUG start site of the mRNA for an essential bacterial protein or a critical rRNA target region, have enhanced anti-bacterial activity as evidence by enhanced inhibition of bacterial growth when they are conjugated to short arginine- and/or lysine-containing peptides. Unconjugated antisense compounds having a ribose or morpholino subunit backbone may be most effective in inhibiting bacterial growth when the subunit length is between 10-12 bases, preferably 11 bases, where the compound contains at least 10 bases, preferably 11-12 bases, that are complementary to the target mRNA sequence. Unconjugated or conjugated antisense compounds having a morpholino subunit backbone containing phosphorodiamidate linkages show further enhancement of anti-bacterial activity with the insertion of cationic 1-piperazine linkages, with the backbone containing at least 1, preferably at least 3 (*e.g.,* 1, 2, 3, 4, 5, 6 positively charged linkages), but no more than half the total number of linkages. These studies were carried out on bacterial gene expression in pure culture, a bacterial cell-free protein expression system and an *in vivo* murine peritonitis model, as described in PCT Application Nos. US05/023553, US06/027522 and US07/011431 incorporated herein by reference in their entirety.

Certain antisense compounds can be conjugated to carrier peptides. More generally, the peptide conjugated to the oligomer in the compound is a peptide of 6-14 residues in length containing at least two, preferably three or more arginine residues. More generally, an "arginine-rich carrier peptide" is one having at least 2, and preferably 3 to 9 arginine residues, each preferably separated by one or more uncharged, hydrophobic residues. In exemplary embodiments, the carrier peptide has the sequence (RXX)ₙ, where X is an uncharged amino acid selected from the group consisting of alanine, valine, leucine, isoleucine, serine, threonine, phenylalanine, tyrosine, and tryptophan, and n= 2 or 3. Also in exemplary embodiments, the carrier peptide has the form (RFF)ₙ or (RFF)ₙR, where n = 2 or 3. In a preferred embodiment, the carrier peptide has the form (RAhxR)₄ The carrier peptide may be linked at its C-terminus to one end of the oligonucleotide, e.g., the 5'- or 3'-end, through a one-or two-amino acid linker, such as the linker is AhxβAla, where Ahx is 6-aminohexanoic acid and βAla is β-alanine, and where the linker forms part of the carrier peptide. One exemplary peptide named (RAhxR)₄ consisting of the sequence RAhxRRAhxRRAhxRRAhxRAhxβAla-COOH (SEQ ID NO: 33) contains 4 repeating Arg-Ahx-Arg sequences. An exemplary conjugate derived from the (RAhxR)₄ peptide (LeuD1 + P007) is listed in Table 3 as SEQ ID NO: 34.

Based on these considerations, exemplary targeting sequences for use in practicing the claimed invention are those having between 10-20 bases, preferably complete but at least 10-base complementarity with the mRNA target sequence, and complementary to a region of the mRNA that includes the AUG start site or a region up to 20 bases downstream of the start site. Exemplary MTb targeting sequences are listed below in Table 1. The antisense compounds provided herein can comprise, consist, or consist essentially of any one or more of these exemplary MTb targeting sequences

**Table 1. Exemplary Mycobacterium tuberculosis antisense targeting sequences**

| **Name** | **Target Gene** | **Sequence (5' - 3')** | **SEQ ID NO.** |
|---|---|---|---|
| leuD1 | leuD | GTG AAA GGC TT | 1 |
| leuD2 | leuD | CTT CCA TGC TG | 2 |
| leuD3 | leuD | CTG ATC CCC TA | 3 |
| mgtC1 | mgtC | GTC AGC GTT TG | 4 |
| mqtC2 | mgtC | TTT GCA TGA CG | 5 |
| mtqC3 | mtgC | ACG CCG AGT CC | 6 |
| pirG | pirG | CGG TTC GGC AC | 7 |
| Rv0194-1 | Rv0194 | GCA ATT CGT GC | 8 |
| Rv0194-2 | Rv0194 | TGC GCA TTC GC | 9 |
| Rv0194-3 | Rv0194 | CGC CAT ATC CT | 10 |
| pcaA1 | pcaA | GAG CTG CAC GG | 11 |
| pcaA2 | pcaA | ACG GAC ATA CC | 12 |
| pcaA3 | pcaA | CCG ATT GAG CC | 13 |
| cma1-1 | cma1 | CAG CTC GTC GG | 14 |
| cma1-2 | cma1 | CGG GCA TTG GG | 15 |
| cma1-3 | cma1 | GGG ATA GCG TA | 16 |
| Rv0477c-1 | Rv0477c Secreted protein | AGG GCT TTC AT | 35 |
| Rv0477c-2 | Rv0477c Secreted protein | CAC CAG GGC TT | 36 |
| Rv2958c-1 | Rv2958c Glycosyl transferase | ATT CCC GCA GC | 37 |
| Rv2958c-2 | Rv2958c Glycosyl transferase | AGC ACA TCG AA | 38 |
| Rv2958c-3 | Rv2958c Glycosyl transferase | GAAA AGT GCG CT | 39 |
| Rv2957-1 | Rv2957 Glycosyl transferase | TTT CGT CTG CA | 40 |
| Rv2957-2 | Rv2957 Glycosyl transferase | GTC TGC ACC AT | 41 |

Any essential bacterial gene can be targeted using the methods of the claimed subject matter. As described above, an essential bacterial gene for any bacterial species can be determined using a variety of methods including those described by Gerdes for E. coli (Gerdes, Scholle et al. 2003). Many essential genes are conserved across the bacterial kingdom thereby providing additional guidance in target selection. Target regions can be obtained using readily available bioinformatics resources such as those maintained by the National Center for Biotechnology Information (NCBI). Complete reference genomic sequences for a large number of microbial species can be obtained including MTb (*e.g*., see http://www.ncbi.nlm.nih.gov/genomes/lproks.cgi) and sequences for essential bacterial genes identified. Bacterial strains can be obtained from the American Type Culture Collection (ATCC). Cell culture methods, such as those described in the Examples, using the appropriate culture medium and conditions for any given species, including MTb, can be established to determine the antibacterial activity of antisense compounds. Once a suitable targeting antisense oligomer has been identified, the peptide moieties of the compounds can be altered to obtain optimal antibacterial activity. An optimal peptide moiety can then be fixed and alternative antisense moieties tested for improved antibacterial activity. One or more iterations of this process can lead to compounds with improved activity but, in general, no more than two iterations are needed to identify highly active antibacterial agents.

### IV. Host gene targets

Another aspect of the invention is the manipulation of host gene expression for the management of MTb infection-induced pathogenesis. In certain embodiments, antisense targeting of selected host genes can be used as a stand-alone therapy for treating MTb infections. In other embodimetns, antisense targeting of selected host genes can be used in combination with MTb-targeted antisense agents, as described herein, and/or other MTb-specific therapies, *e.g*., antibiotics. The antisense targeting of certain genes involved in immune suppression via the IL-10 signaling pathway for the control of MTb infection has been previously described (*see*, *e.g.*, PCT Application No. US08/088339 herein incorporated by reference in its entirety). It is an objective of the present invention to extend this approach to genes encoding STAT3, IL-27 and TGF-beta, and SOCS1, among others. Exemplary antisense targeting sequences to these genes and those involved in the IL-10 signaling pathway (IL-10, SOCS1 and SOCS3) are listed below in Table 2 as SEQ ID NOs: 17-23. These targeting sequences in Table 2 are complementary to the translation initiation region of the host genes. Also included are targeting sequences that are complementary to splice acceptor or splice donor sites of the pre-mRNA molecule of a selected host gene, including those that are complementary to a region containing the 50 bases surrounding the splice donor or splice acceptor sites.

**Table 2. Host Gene Targeting Sequences**

| **Name** | **Sequence (5'-3')** | **SEQ ID NO** |
|---|---|---|
| IL-10E4SA | GGAGAAATCGATGCTGAAGAA | 17 |
| SOCS1 | CTGGTTGCGTGCTACCATCC | 18 |
| SOCS3 | GCCGCTACCGCATCCCIGIGA | 19 |
| STAT3 | TGGCTTCTCAAGATACCTGCT | 20 |
| IL-27-AUG | GTCACCTGGCCCATGTCAGG | 21 |
| IL-27E3SA | CAATCGAGATTCAGCCTGAG | 22 |
| TGF-beta | GAGGGCGGCATGIIIGAGGC | 23 |

### V. Method for inhibiting MTb

In one aspect, the claimed subject matter includes a method of inhibiting an MTb infection, by exposing the infecting bacteria to a 10-20 base oligomeric antisense compound of the type characterized herein. These antisense compounds can be targeted against one or more MTb genes (*e.g*., *rpsJ, leuD*, *mgtC, pirG*, *pcaA*, *cma1*, *Rv0194, Rv0477c*, *Rv2958c,* and *Rv2957*), and/or one or more selected host genes (*e.g*., STAT3, IL-27 and TGF-beta, and SOCS1). In one aspect, the method is applied to inhibiting a bacterial infection in a mammalian subject, including a human subject, by administering the antisense compound to the subject in a therapeutic amount.

In certain embodiments, an improved antibacterial PMO can be obtained by conjugating a short 6-12 amino acid peptide that enhances either intracellular delivery or antisense activity or both. Exemplary peptides and peptide-conjugated PMO (PPMO) are listed in Table 3 as SEQ ID NOS: 24 - 34.

**Table 3. Peptide and Peptide-PMO Sequences**

| **Name** | **Sequence** | **SEQ ID NO** |
|---|---|---|
| (RFF)₃; CP04073 | N-RFFRFFRFFAhxβAla-COOH | 24 |
| RTR | N-RTRTRFLRRTAhxβAla-COOH | 25 |
| RFFR | N-RFFRFFRFFRAhxβAla-COOH | 26 |
| KTR | N-KTRTKFLKKTAhxβAla-COOH | 27 |
| KFF | N-KFFKFFKFFAhxβAla-COOH | 28 |
| KFFK | N-KFFKFFKFFKAhxβAla-COOH | 29 |
| (RFF)₂ | N-RFFRFFAhxβAla-COOH | 30 |
| (RFF)₂R | N-RFFRFFRAhxβAla-COOH | 31 |
| RAhx | N-RAhxAhxRAhxAhxRAhxAhxβAla -COOH | 32 |
| (RAhxR)₄; P007 | N-RAhxRRAhxRRAhxRRAhxRAhxβAla -COOH | 33 |
| P007leuD1 | 5'-GTGAAAGGCTT-bAlaAhxRAhxRRAhxRRAhxRRAhxR-N | 34 |

It will be understood that the *in vivo* efficacy of such a PMO in a subject using the methods of the claimed invention is dependent upon numerous factors including, but not limited to, (1) the target sequence; (2) the duration, dose and frequency of antisense administration; and (3) the general condition of the subject.

The efficacy of an *in vivo* administered antisense oligomer of the claimed subject matter in inhibiting or eliminating the growth of one or more types of bacteria may be determined by *in vitro* culture or microscopic examination of a biological sample (tissue, blood, *etc*.) taken from a subject prior to, during and subsequent to administration of the PMO. (See, for example, (Pari, Field et al. 1995); and (Anderson, Fox et al. 1996).

### A. Administration Methods

Effective delivery of the anti-MTb compounds of the invention to the target nucleic acid is an important aspect of treatment. In accordance with the claimed invention, such routes of delivery include, but are not limited to, various systemic routes, including oral and parenteral routes, *e.g*., intravenous, subcutaneous, intraperitoneal, and intramuscular, as well as inhalation, transdermal and topical delivery. The appropriate route may be determined by one of skill in the art, as appropriate to the condition of the subject under treatment. For example, an appropriate route for delivery of an anti-MTb compound in the treatment of a MTb respiratory infection is by inhalation. Methods effective to deliver the oligomer to the site of MTb infection or to introduce the compound into the bloodstream are also contemplated.

Transdermal delivery of an anti-MTb compound may be accomplished by use of a pharmaceutically acceptable carrier adapted for topical administration. One example of morpholino oligomer delivery is described in PCT Application No. US09/068599, incorporated herein by reference.

In one preferred embodiment, the anti-MTb compound is a P-PMO, contained in a pharmaceutically acceptable carrier, and is delivered orally.

The anti-MTb compound may be administered in any convenient vehicle which is physiologically acceptable. Such a composition may include any of a variety of standard pharmaceutically accepted carriers employed by those of ordinary skill in the art. Examples of such pharmaceutical carriers include, but are not limited to, saline, phosphate buffered saline (PBS), water, aqueous ethanol, emulsions such as oil/water emulsions, triglyceride emulsions, wetting agents, tablets and capsules. It will be understood that the choice of suitable physiologically acceptable carrier will vary dependent upon the chosen mode of administration.

In some instances liposomes may be employed to facilitate uptake of the antisense oligonucleotide into cells. (See, *e.g*., Williams, S.A., Leukemia 10(12):1980-1989, 1996; Lappalainen et al., Antiviral Res. 23:119, 1994; Uhlmann et a/., ANTISENSE OLIGONUCLEOTIDES: A NEW THERAPEUTIC PRINCIPLES, Chemical Reviews, Volume 90, No. 4, pages 544-584, 1990; Gregoriadis, G., Chapter 14, Liposomes, Drug Carriers in Biology and Medicine, pp. 287-341, Academic Press, 1979). Hydrogels may also be used as vehicles for antisense oligomer administration, for example, as described in WO 93/01286. Alternatively, the oligonucleotides may be administered in microspheres or microparticles. (See, *e.g.,* Wu, G.Y. and Wu, C.H., J. Biol. Chem. 262:4429-4432, 1987.)

Sustained release compositions are also contemplated within the scope of this application. These may include semipermeable polymeric matrices in the form of shaped articles such as films or microcapsules.

Typically, one or more doses of the anti-MTb compound are administered, generally at regular intervals for a period of about one to two weeks. Preferred doses for oral administration are from about 10 mg oligomer/patient to about 1000 mg oligomer/patient (based on a weight of 70 kg). In some cases, doses of greater than 250 mg oligomer/patient may be necessary. For IV administration, the preferred doses are from about 1.0 mg oligomer/patient to about 1000 mg oligomer/patient (based on an adult weight of 70 kg). The anti-MTb compound is generally administered in an amount and manner effective to result in a peak blood concentration of at least 200-400 nM oligomer.

In a further aspect of this embodiment, an anti-MTb compound is administered at regular intervals for a short time period, *e.g*., daily for two weeks or less. However, in some cases the anti-MTb compound is administered intermittently over a longer period of time. Administration of an anti-MTb compound to a subject may also be followed by, or concurrent with, administration of an antibiotic or other therapeutic treatment.

In one aspect of the method, the subject is a human subject, *e.g*., a patient diagnosed as having a localized or systemic MTb infection. The condition of a patient may also dictate prophylactic administration of an anti-MTb compound of the claimed subject matter, *e.g.* in the case of a patient who (1) is immunocompromised; (2) is a burn victim; (3) has an indwelling catheter; or (4) is about to undergo or has recently undergone surgery.

The methods of the invention are applicable, in general, to treatment of any condition wherein inhibiting or eliminating the growth of MTb would be effective to result in an improved therapeutic outcome for the subject under treatment.

One aspect of the invention is a method for treatment of a bacterial infection which includes the administration of an anti-MTb morpholino antisense oligomer to a subject, followed by or concurrent with administration of an antibiotic or other therapeutic treatment to the subject.

### B. Treatment Monitoring Methods

It will be understood that an effective *in vivo* treatment regimen using the anti-MTb compounds of the invention will vary according to the frequency and route of administration, as well as the condition of the subject under treatment (*i.e.*, prophylactic administration versus administration in response to localized or systemic infection). Accordingly, such *in vivo* therapy will generally require monitoring by tests appropriate to the particular type of bacterial infection under treatment and a corresponding adjustment in the dose or treatment regimen in order to achieve an optimal therapeutic outcome.

The efficacy of a given therapeutic regimen involving the methods described herein may be monitored, *e.g*., by general indicators of MTb infection, such as complete blood count (CBC), nucleic acid detection methods, immunodiagnostic tests, or bacterial culture.

Identification and monitoring of MTb infection generally involves one or more of (1) nucleic acid detection methods, (2) serological detection methods, *i.e.,* conventional immunoassay, (3) culture methods, and (4) biochemical methods. Such methods may be qualitative or quantitative.

Nucleic acid probes may be designed based on publicly available bacterial nucleic acid sequences, and used to detect target genes or metabolites (*i.e.*, toxins) indicative of bacterial infection, which may be specific to a particular bacterial type, *e.g.*, a particular species or strain, or common to more than one species or type of bacteria (*i.e.*, Gram positive or Gram negative bacteria). Nucleic amplification tests (*e.g*., PCR) may also be used in such detection methods.

Serological identification may be accomplished using a bacterial sample or culture isolated from a biological specimen, *e.g*., stool, urine, cerebrospinal fluid, blood, *etc.* Immunoassay for the detection of bacteria is generally carried out by methods routinely employed by those of skill in the art, *e.g*., ELISA or Western blot. In addition, monoclonal antibodies specific to particular bacterial strains or species are often commercially available.

Culture methods may be used to isolate and identify particular types of bacteria, by employing techniques including, but not limited to, aerobic versus anaerobic culture, growth and morphology under various culture conditions. Exemplary biochemical tests include Gram stain (Gram, 1884; Gram positive bacteria stain dark blue, and Gram negative stain red), enzymatic analyses (*i.e*., oxidase, catalase positive for *Pseudomonas aeruginosa*), and phage typing.

It will be understood that the exact nature of such diagnostic, and quantitative tests as well as other physiological factors indicative of bacterial infection will vary dependent upon the bacterial target, the condition being treated and whether the treatment is prophylactic or therapeutic.

In cases where the subject has been diagnosed as having a particular type of bacterial infection, the status of the bacterial infection is also monitored using diagnostic techniques typically used by those of skill in the art to monitor the particular type of bacterial infection under treatment.

The P-PMO+ treatment regimen may be adjusted (dose, frequency, route, *etc*.), as indicated, based on the results of immunoassays, other biochemical tests and physiological examination of the subject under treatment.

From the foregoing, it will be appreciated how various objects and features of the invention are met. The method provides an improvement in therapy against MTb infection, using anti-MTb compounds to achieve enhanced cell uptake and anti-bacterial action. As a result, drug therapy is more effective and less expensive, both in terms of cost and amount of compound required.

An important advantage of the invention is that compounds effective against virtually any pathogenic MTb can be readily designed and tested, *e.g*., for rapid response against new drug-resistant MTb.

The following examples are intended to illustrate but not to limit the invention. Each of the patent and non-patent references referred to herein are incorporated by reference in their entirety.

### Materials and Methods

### Phosphorodiamidate Morpholino Oligomers

PMO were synthesized and purified at AVI BioPharma, Inc. (Corvallis, OR) as previously described (Summerton and Weller 1997; Geller, Deere et al. 2003) and further described in pending applications US#12/271,036, US#12/271,040 and PCT publication number WO/2009/064471. Sequences of exemplary PMO of the present invention are shown in Tables 1 and 2 and listed below in the Sequence Listing. The concentration of PMO was determined spectrophotometrically by measuring the absorbance at 260 nm and calculating the molarity using the appropriate extinction coefficient.

### Peptide synthesis and conjugation to PMO

Peptide synthesis of (RFF)₃XB (CP04073; (RFF)₃AhxβAla), and (RXR)₄XB (P007; RXRRXRRXRRXRAhxβAla), (SEQ ID NOs: 24 and 23, respectively) and conjugation to PMO were performed as described in PCT Application No. US05/018213. All the peptides of the present invention can be synthesized and conjugated to PMO using these synthetic techniques.

### Oligomer Sequences

Exemplary targeting oligomers used in describing the present invention are listed below the Sequence Listing. The listed oligomers all target laboratory strains of MTb or murine host genes and used in experiments in support of the invention. The Sequence Listing also lists the peptides of the that can be conjugated to any of the PMO, see above, to form PPMO conjugates and also used in experiments in support of the invention.

### EXAMPLE 1:

### EVALUATION OF PMO COMPOUNDS IN MACROPHAGE CULTURES INFECTED WITH M. TUBERCULOSIS

This example describes the evaluation of the compounds listed in Table 1 in macrophage cultures infected with laboratory strains of *M tuberculosis.* Cultures are infected then treated with the test comopunds at concentrations ranging from 0.01 to 10.0 uM. The test compounds are added to the growth medium from a stock solution (1mM) and remain in the cultures during the duration of the evaluation period. Given the relatively slow growth rate of *M tuberculosis,* longer incubations can require multiple additions of the oligomers. The operating protocols are adjusted based on preliminary observations. The critical endpoints of the studies include macrophage survival and reduction in the growth of *M*. *tuberculosis.* A satisfactory magnitude of response is determined based on initial observations.

### EXAMPLE 2:

### EVALUATION OF EFFECTIVE COMPOUNDS FROM EXAMPLE 1 IN A MOUSE MODEL OF TB

Lead candidates from the cell culture screen from Example 1 are evaluated in a mouse model with the laboratory strains of *M*. *tuberculosis.* The mice are infected then treated with the lead test compound. The dose range is from 30 ug/mouse to 300µg/mouse (based on prior experience with PPMO efficacy and toxicity). The route of administration may include intraperitoneal, intravenous and insufflation. The dose interval is from +0.5 to 2 hours post-infection and at 12 hour intervals for up to two weeks. Initial studies will utilize fewer mice per group (*e.g*., 3-4) but confirmatory studies will require larger numbers of mice per group (*e.g*., 8-12). The critical endpoints of the studies will include survival, changes in body weight and reduction in the growth of *M tuberculosis.*

### EXAMPLE 3:

### EXPLORE HOST-DIRECTED AGENTS IN A MOUSE MODEL OF TB INFECTION

The utility of host directed agents are not easily observed in cell culture due to the lack of complex cell-to-cell communication and an appropriate cellular environmental context. Hence, these studies are conducted in a mouse model infected with the laboratory strain of *M tuberculosis.* The compounds tested are listed in Table 3. The mice are infected then treated with the lead compounds. The dose range is from 30 microgram to 300 microgram per mouse. The route of administration include intraperitoneal, intravenous and insufflation. The dose interval is from +0.5 to 2 hours post-infection and at 12 hour intervals for up to two weeks. Initial studies utilize fewer mice per group (*e.g*., 3-4) and confirmatory studies require larger numbers of mice per group (*e.g*., 8-12). The critical endpoints of the studies include survival, changes in body weight and reduction in the growth of *M tuberculosis* as measured by serum colony forming units.

**Sequence Listing:**

| **Name** | **Sequence (5' to 3' or amino to carboxyl)** | **SEQ ID NO.** |
|---|---|---|
| leuD1 | GTG AAA GGC TT | 1 |
| leuD2 | CTT CCA TGC TG | 2 |
| leuD3 | CTG ATC CCC TA | 3 |
| mgtC1 | GTC AGC GTT TG | 4 |
| mgtC2 | TTT GCA TGA CG | 5 |
| mtgC3 | ACG CCG AGT CC | 6 |
| pirG | CGG TTC GGC AC | 7 |
| Rv0194-1 | GCA ATT CGT GC | 8 |
| Rv0194-2 | TGC GCA TTC GC | 9 |
| Rv0194-3 | CGC CAT ATC CT | 10 |
| pcaA1 | GAG CTG CAC GG | 11 |
| pcaA2 | ACG GAC ATA CC | 12 |
| pcaA3 | CCG ATT GAG CC | 13 |
| cma1-1 | CAG CTC GTC GG | 14 |
| cma1-2 | CGG GCA TTG GG | 15 |
| cma1-3 | GGG ATA GCG TA | 16 |
| II-10E4SA | GGAGAAATCGATGCTGAAGAA | 17 |
| SOCS1 | CTGGTTGCGTGCTACCATCC | 18 |
| SOCS3 | GCCGCTACCGCATCCCIGIGA | 19 |
| STAT3 | TGGCTTCTCAAGATACCTGCT | 20 |
| IL-27-AUG | GTCACCTGGCCCATGTCAGG | 21 |
| IL-27E3SA | CAATCGAGATTCAGCCTGAG | 22 |
| TGFbeta | GAGGGCGGCATGIIIGAGGC | 23 |
| (RFF)₃;CP04073 | N-RFFRFFRFFAhxβAla-COOH | 24 |
| RTR | N-RTRTRFLRRTAhxβAla-COOH | 25 |
| RFFR | N-RFFRFFRFFRAhxβAla-COOH | 26 |
| KTR | N-KTRTKFLKKTAhxβAla-COOH | 27 |
| KFF | N-KFFKFFKFFAhxβAla-COOH | 28 |
| KFFK | N-KFFKFFKFFKAhxβAla-COOH | 29 |
| (RFF)₂ | N-RFFRFFAhxβAla-COOH | 30 |
| (RFF)₂R | N-RFFRFFRAhxβAla-COOH | 31 |
| RAhx | N-RAhxAhxRAhxAhxRAhxAhxβAla -COOH | 32 |
| (RAhxR)₄;P007 | N-RAhxRRAhxRRAhxRRAhxRAhxβAla - COOH | 33 |
| leuD1-P007 | 5'-GTGAAAGGCTT-βAlaAhxRAhxRRAhxRRAhxRRAhxR-N | 34 |
| Rv0477c-1 | AGG GCT TTC AT | 35 |
| Rv0477c-2 | CAC CAG GGC TT | 36 |
| Rv2958c-1 | ATT CCC GCA GC | 37 |
| Rv2958c-2 | AGC ACA TCG AA | 38 |
| Rv2958c-3 | GAAA AGT GCG CT | 39 |
| Rv2957-1 | TTT CGT CTG CA | 40 |
| Rv2957-2 | GTC TGC ACC AT | 41 |

### References:

Anderson, K. P., M. C. Fox, et al. (1996). "Inhibition of human cytomegalovirus immediate-early gene expression by an antisense oligonucleotide complementary to immediate-early RNA." Antimicrob Agents Chemother 40(9): 2004-11.
Blommers, M. J., U. Pieles, et al. (1994). "An approach to the structure determination of nucleic acid analogues hybridized to RNA. NMR studies of a duplex between 2'-OMe RNA and an oligonucleotide containing a single amide backbone modification." Nucleic Acids Res 22(20): 4187-94.
Bramhill, D. (1997). "Bacterial cell division." Annu Rev Cell Dev Biol 13: 395-424.
Cross, C. W., J. S. Rice, et al. (1997). "Solution structure of an RNA x DNA hybrid duplex containing a 3'-thioformacetal linker and an RNA A-tract." Biochemistry 36(14): 4096-107.
Donachie, W. D. (1993). "The cell cycle of Escherichia coli." Annu Rev Microbiol 47: 199-230.
Gait, M. J., A. S. Jones, et al. (1974). "Synthetic-analogues of polynucleotides XII. Synthesis of thymidine derivatives containing an oxyacetamido- or an oxyformamido-linkage instead of a phosphodiester group." J Chem Soc [Perkin 1] 0(14): 1684-6.
Geller, B. L., J. D. Deere, et al. (2003). "Inhibition of gene expression in Escherichia coli by antisense phosphorodiamidate morpholino oligomers." Antimicrob Agents Chemother 47(10): 3233-9.
Geller, B. L. and H. M. Green (1989). "Translocation of pro-OmpA across inner membrane vesicles of Escherichia coli occurs in two consecutive energetically distinct steps." J Biol Chem 264(28): 16465-9.
Gerdes, S. Y., M. D. Scholle, et al. (2003). "Experimental determination and system level analysis of essential genes in Escherichia coli MG1655." J Bacteriol 185(19): 5673-84.
Good, L., S. K. Awasthi, et al. (2001). "Bactericidal antisense effects of peptide-PNA conjugates." Nat Biotechnol 19(4): 360-4.
Good, L. and P. E. Nielsen (1998). "Inhibition of translation and bacterial growth by peptide nucleic acid targeted to ribosomal RNA." Proc Natl Acad Sci U S A 95(5): 2073-6.
Greenberg, D. E., K. R. Marshall-Batty, et al. (2010). "Antisense Phosphorodiamidate Morpholino Oligomers Targeted to an Essential Gene Inhibit Burkholderia cepacia Complex." J Infect Dis.
Hale, C. A. and P. A. de Boer (1999). "Recruitment of ZipA to the septal ring of Escherichia coli is dependent on FtsZ and independent of FtsA." J Bacteriol 181(1): 167-76.
Lesnikowski, Z. J., M. Jaworska, et al. (1990). "Octa(thymidine methanephosphonates) of partially defined stereochemistry: synthesis and effect of chirality at phosphorus on binding to pentadecadeoxyriboadenylic acid." Nucleic Acids Res 18(8): 2109-15.
Lutkenhaus, J. and S. G. Addinall (1997). "Bacterial cell division and the Z ring." Annu Rev Biochem 66: 93-116.
Mertes, M. P. and E. A. Coats (1969). "Synthesis of carbonate analogs of dinucleosides. 3'-Thymidinyl 5'-thymidinyl carbonate, 3'-thymidinyl 5'-(5-fluoro-2'-deoxyuridinyl) carbonate, and 3'-(5-fluoro-2'-deoxyuridinyl) 5'-thymidinyl carbonate." J Med Chem 12(1): 154-7.
Pari, G. S., A. K. Field, et al. (1995). "Potent antiviral activity of an antisense oligonucleotide complementary to the intron-exon boundary of human cytomegalovirus genes UL36 and UL37." Antimicrob Agents Chemother 39(5): 1157-61.
Rahman, M. A., J. Summerton, et al. (1991). "Antibacterial activity and inhibition of protein synthesis in Escherichia coli by antisense DNA analogs." Antisense Res Dev 1(4): 319-27.
Summerton, J., D. Stein, et al. (1997). "Morpholino and phosphorothioate antisense oligomers compared in cell-free and in-cell systems." Antisense Nucleic Acid Drug Dev 7(2): 63-70.
Summerton, J. and D. Weller (1997). "Morpholino antisense oligomers: design, preparation, and properties." Antisense Nucleic Acid Drug Dev 7(3): 187-95.
Zhang, Y. and J. E. Cronan, Jr. (1996). "Polar allele duplication for transcriptional analysis of consecutive essential genes: application to a cluster of Escherichia coli fatty acid biosynthetic genes." J Bacteriol 178(12): 3614-20.

The present invention also relates to the following items:
1. A substantially uncharged antisense morpholino oligonucleotide, composed of morpholino subunits and phosphorus-containing intersubunit linkages joining a morpholino nitrogen of one subunit to a 5'-exocyclic carbon of an adjacent subunit, and having
   a) 10-20 nucleotide bases;
   b) a targeting sequence of at least 10 bases in length directed against a bacterial mRNA that encodes an essential protein from *Mycobacterium tuberculosis* (MTb), wherein said targeting sequence is complementary to a target sequence containing the translational start codon of said bacterial mRNA or to a target sequence containing 20 bases downstream of the translational start codon of said bacterial mRNA; and
   c) a Tm, when hybridized to the target sequence, of between about 45°C to 65°C.
2. The oligonucleotide of item 1, wherein the morpholino subunits are joined by phosphorodiamidate linkages in accordance with the structure: 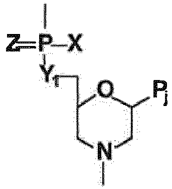 where Y₁=O, Z=O, Pj is a purine or pyrimidine base-pairing moiety effective to bind, by base-specific hydrogen bonding, to a base in a polynucleotide, and X is alkyl, alkoxy, thioalkoxy, or alkyl amino.
3. The oligonucleotide of item 2, in which at least 2 and no more than half of the total number of intersubunit linkages are positively charged.
4. The oligonucleotide of item 2 or 3, wherein X=NR₂, and wherein each R is independently hydrogen or methyl, and for the positively charged linkages, X is 1-piperazine.
5. The oligonucleotide of any one of items 1-4, wherein the essential MTb protein is selected from *rpsJ*, *leuD*, *mgtC, pirG*, *pcaA*, *cma1* and *Rv0194*, *Rv0477c*, *Rv2958c*, *Rv2957*, *gyrA,* and *acpP.*
6. The oligonucleotide of item 5, wherein the targeting sequence comprises the nucleotide sequence set forth in at least one of SEQ ID NOS:1-16 or 35-41.
7. The oligonucleotide of any one of items 1-6, which is conjugated to an arginine-rich polypeptide that enhances the uptake of the compound into host cells.
8. The oligonucleotide of item 7, wherein the arginine-rich polypeptide is selected from the group consisting of SEQ ID NOS:24-34.
9. A method of reducing replication of *Mycobacterium tuberculosis* (MTb), comprising contacting an MTb bacteria or an MTb-infected host cell with an antisense morpholino oligonucleotide of any one of items 1-8.
10. The method of item 9, wherein the bacteria or host cell is in a subject, and the method comprises administering the antisense oligonucleotide to the subject.
11. The method of item 9 or 10, wherein the MTb is a multidrug-resistant MTb (MDR-MTb) or an extensively-drug resistant MTb (XDR-MTb).
12. A method of reducing replication of *Mycobacterium tuberculosis* (MTb), comprising contacting an MTb-infected host cell with an antisense morpholino oligonucleotide, composed of morpholino subunits and phosphorus-containing intersubunit linkages joining a morpholino nitrogen of one subunit to a 5'-exocyclic carbon of an adjacent subunit, and having
   a) 10-20 nucleotide bases,
   b) a targeting sequence of at least 10 bases in length directed against a mammalian host mRNA that encodes IL-10, SOCS1, SOCS3, STAT3, IL-27, or TGF-beta, wherein said targeting sequence is complementary to (i) a target sequence containing the translational start codon of said mRNA, (ii) a target sequence containing 20 bases downstream of the translational start codon of said mRNA, or (iii) a target sequence containing the 50 bases surrounding a splice donor or a splice acceptor site of said mRNA; and
   c) a Tm, when hybridized to the target sequence, of between about 45°C to 65°C.
13. The method of item 12, wherein the targeting sequence comprises the nucleotide sequence set forth in at least one of SEQ ID NOS: 17-23.
14. The method of item 12 or 13, wherein the oligonucleotide is conjugated to an arginine-rich polypeptide that enhances the uptake of the compound into host cells.
15. The method of item 14, wherein the arginine-rich polypeptide is selected from the group consisting of SEQ ID NOS:24-34.
16. The method of any one of items 12-15, wherein the host cell is in a subject, and the method comprises administering the antisense oligonucleotide to the subject.
17. The method of item 16, further comprising administering separately or concurrently an anti-MTb antibiotic, an antisense oligonucleotide of any one of items 1-8, or both.
18. The method of item 17, wherein the anti-MTb antibiotic is selected from at least one of ethambutol, isoniazid, pyrazinamide, rifampicin, streptomycin, amikacin, kanamycin, capreomycin, viomycin, enviomycin, ciprofloxacin, levofloxacin, moxifloxacin, ethioniamid, prothionamide, cycloserine, p-aminosalicylic acid, rifabutin, clarithromycin, linezolid, thioacetamide, thioacetazone, and thioridazine.
19. A pharmaceutical composition comprising an antisense oligonucleotide of any one of items 1-8, and a pharmaceutically acceptable carrier.
20. The pharmaceutical composiition of item 18, further comprising an antisense oligonucleotide of any one of items 12-15.

## Claims

1. A substantially uncharged antisense morpholino oligonucleotide, composed of morpholino subunits and phosphorus-containing intersubunit linkages joining a morpholino nitrogen of one subunit to a 5'-exocyclic carbon of an adjacent subunit, in which between 50-100% of the subunit linkages are uncharged at physiological pH, and contain a single phosphorous atom, and having
a) 10-20 bases;
b) a targeting sequence of at least 10 bases in length directed against a bacterial mRNA that encodes an essential protein from *Mycobacterium tuberculosis* (MTb), wherein said targeting sequence is complementary to a target sequence containing the translational start codon of said bacterial mRNA or to a target sequence containing 20 bases downstream of the translational start codon of said bacterial mRNA, wherein said essential protein is pirG; and
c) a Tm, when hybridized to the target sequence, of 45°C to 65°C.

2. The oligonucleotide of claim 1, wherein the morpholino subunits are joined by phosphorus-containing intersubunit linkages in accordance with the structure: 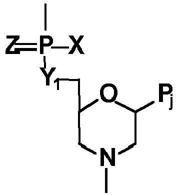 where Y₁=O, Z=O, Pj is a purine or pyrimidine base-pairing moiety effective to bind, by base-specific hydrogen bonding, to a base in a polynucleotide, and X is alkyl, alkoxy, thioalkoxy, or alkyl amino.

3. The oligonucleotide of claim 2, in which at least 2 and no more than half of the total number of phosphorus-containing intersubunit linkages are positively charged.

4. The oligonucleotide of claim 2 or 3, wherein X=NR₂, and wherein each R is independently hydrogen or methyl, and for the positively charged linkages, X is 1-piperazine.

5. The oligonucleotide of any one of claims 1-4, which is conjugated to an arginine-rich peptide that enhances the uptake of the compound into host cells.

6. The oligonucleotide of claim 5, wherein the arginine-rich peptide is selected from the group consisting of SEQ ID NOS:24-34.

7. An *in vitro* method of reducing replication of *Mycobacterium tuberculosis* (MTb), comprising contacting an MTb bacteria or an MTb-infected host cell with an antisense morpholino oligonucleotide of any one of claims 1-6.

8. The method of claim 7, wherein the bacteria or host cell is in a subject, and the method comprises administering the antisense oligonucleotide to the subject.

9. The method of claim 7 or 8, wherein the MTb is a multidrug-resistant MTb (MDR-MTb) or an extensively-drug resistant MTb (XDR-MTb).

10. An antisense morpholino oligonucleotide of any one of claims 1-6 for reducing replication of *Mycobacterium tuberculosis* (MTb), comprising administering the antisense oligonucleotide to a subject having an MTb bacteria or an MTb-infected host cell.

11. The antisense morpholino oligonucleotide of any one of claims 1-6 for the use of claim 10, wherein the MTb is a multidrug-resistant MTb (MDR-MTb) or an extensively-drug resistant MTb (XDR-MTb).

12. A pharmaceutical composition comprising an antisense oligonucleotide of any one of claims 1-6, and a pharmaceutically acceptable carrier.
